# EUROPÄISCHE PATENTSCHRIFT

(11) **EP 1 854 412 B1**
(45) Veröffentlichungstag und Bekanntmachung des Hinweises auf die Patenterteilung: **06.01.2010**
(21) Anmeldenummer: 07007789.6
(22) Anmeldetag: 17.04.2007
(51) Int. Cl.: A61B 6/08, A61N 5/10

(54) **Überprüfung der Ausrichtung von Lasern an einer Diagnose- und/oder Therapiemaschine**
Verification of the alignment of lasers on a diagnostic and/or therapy machine
Surveillance du positionnement de lasers sur un appareil de diagnostic et/ou de thérapie

(30) Priorität: 10.05.2006 DE 102006021681
(43) Veröffentlichungstag der Anmeldung: 14.11.2007
(73) Patentinhaber: LAP GmbH Laser Applikationen, D-21337 Lüneburg (DE)
(72) Erfinder: Röckseisen, Armin, Dr., 21379 Scharnebeck (DE)
(74) Vertreter: Schildberg, Peter

(56) Entgegenhaltungen:
- EP-A1- 0 753 285
- WO-A-02/100477
- WO-A-2006/055770
- DE-A1- 19 907 065
- GB-A- 2 317 545
- US-A- 5 142 559

## Beschreibung

Die vorliegende Erfindung betrifft eine Vorrichtung und ein Verfahren zur Überprüfung der Ausrichtung von Laserstrahlen an einer Diagnose- und/oder Therapiemaschine, wobei die Laserstrahlen zur Anzeige einer Behandlungsposition relativ zu einem Behandlungsstrahl dienen.

In der Strahlentherapie werden verschiedene bildgebende diagnostische Verfahren eingesetzt, um einen Patienten näher zu untersuchen. Bildgebende Verfahren werden insbesondere bei einem Verdacht auf eine Tumorerkrankung eingesetzt, um Ort, Größe sowie Lage des Tumors im Körper des Patienten bildlich darzustellen. Für eine spätere Bestrahlung des Tumors wird zu einem späteren Zeitpunkt ein Therapiegerät eingesetzt, was eine Übertragung der diagnostischen Ergebnisse auf das Therapiegerät erforderlich macht. Wird bei dem bildgebenden Verfahren also die Lage des Tumors bestimmt, so ist es erforderlich, daß der Patient zu einem späteren Zeitpunkt relativ zum Therapiegerät so ausgerichtet wird, daß der Behandlungsstrahl durch den Tumor verläuft. Bei einem verfahrbaren Behandlungsstrahl, der aus unterschiedlichen Richtungen auf das zu behandelnde Gebiet fällt, ist für die richtige Ausrichtung des Patienten darauf zu achten, daß das Isozentrum des Behandlungsstrahls sich in der vorbestimmten Position befindet. Bei einer Diagnosemaschine gibt es kein maschinenbedingtes Isozentrum, sondern einen anderen maschinentypabhängigen mechanischen Bezugspunkt, ein sogenanntes virtuelles Isozentrum. Bei einem Computertomographen wird beispielsweise das virtuelle Isozentrum auf der Rotationsachse definiert. Im nachfolgenden Text wird nicht zwischen Isozentrum der Behandlungsmaschine und virtuellem Isozentrum der Diagnosemaschine unterschieden.

Um den Patienten an der Maschine in eine vorbestimmte Position zu bringen, so daß beispielsweise das zu behandelnde Gewebegebiet exakt relativ zu dem Behandlungsstrahl ausgerichtet ist, werden bei dem Diagnoseverfahren Markierungen auf der Haut des Patienten aufgebracht. Die Stelle für die Markierungen werden beispielsweise durch Laser an der Maschine, die das bildgebende Verfahren ausführt, auf die Haut des Patienten projiziert. Die Markierungen können beispielsweise mit einem Stift auf die Haut des Patienten übertragen werden. Auch ist es möglich, Marker auf die Haut zu kleben. So können beispielsweise die Laserstrahlen auf einen Punkt gerichtet werden, der als späteres Isozentrum vorgesehen ist. Befindet sich der Patient, beispielsweise einige Tage später, an der Therapiemaschine so wird er anhand der Markierungen an der Therapiemaschine in eine Position ausgerichtet, die eine Bestimmung des Behandlungsgebietes erlaubt. Um eine exakte Ausrichtung des Patienten vorzunehmen, werden an der Therapiemaschine ebenfalls wieder in einer genau definierten Weise Laserstrahlen auf die Haut des Patienten projiziert. Befinden sich die aufgebrachten Marker in Übereinstimmung mit den auf die Haut projizierten Laserstrahlen, so befindet sich der Patient wieder in der definierten Position, so daß für die Steuerung der Therapiemaschine anhand der Diagnosedaten genau Größe, Lage und Ausrichtung des zu behandelnden Gewebegebiets vorliegt. Bei der Ausrichtung des Patienten kann, wenn die Laserstrahlen an der Diagnosemaschine auf das vorgesehene Isozentrum gerichtet sind, auch an der Therapiemaschine der Patient wieder auf das Isozentrum des Behandlungsstrahls ausgerichtet werden.

US 5, 142, 559 beschreibt eine Messvorrichtung zum überprüfen der Ausrichtung einer Strahlungsquelle. Die Ausrichtung wird mittels eines Laserstrahls und einer Kamera überprüft. Die Messvorrichtung und die Kamera sind jeweils auf einer Patientenliege befestigbar. Die Ausrichtung des Laserstrahls kann überprüft werden, indem detektiert wird, auf welche Detektoren der Messvorrichtung der Laserstrahl trifft.

Der vorliegenden Erfindung liegt die Aufgabe zugrunde, an einer Therapiemaschine zu prüfen, ob die Laserstrahlen zur Markierung korrekt relativ zu dem Behandlungsstrahl ausgerichtet sind.

Die vorstehende Aufgabe wird durch eine Vorrichtung mit den Merkmalen gemäß Anspruch 1 und durch ein Verfahren mit den Merkmalen gemäß Anspruch 10 gelöst. Vorteilhafte Ausgestaltungen bilden die Gegenstände der Unteransprüche.

Die erfindungsgemäße Vorrichtung dient zur Überprüfung der Ausrichtung von Laserstrahlen zur Anzeige einer Behandlungsposition relativ zu einem Behandlungsstrahl an einer Diagnose- und/oder einer Therapiemaschine. Die Maschine besitzt eine verstellbare Patientenunterlage, deren Position relativ zum Behandlungsstrahl einstellbar ist. Für eine ordnungsgemäße Positionierung des Patienten relativ zum Behandlungsstrahl sind die Laserstrahlen und der Behandlungsstrahl relativ zueinander ausgerichtet derart, daß die Laserstrahlen sich in einem Isozentrum des Behandlungsstrahls schneiden. Bei einer Diagnosemaschine schneiden die Laserstrahlen sich ebenfalls in dem Isozentrum. Die erfindungsgemäße Vorrichtung weist drei Komponenten auf. Ein Trägerelement ist vorgesehen, um in einer vorbestimmten Position an der Patientenunterlage der Maschine angeordnet zu werden. Das Trägerelement ist hierzu mit Mitteln versehen, die eine reproduzierbare und definierte Positionierung auf der Patientenunterlage der Maschine gestatten. Ferner ist eine erste und eine zweite Meßeinrichtung vorgesehen, die jeweils mit Verbindungsmitteln für das Trägerelement ausgestattet sind. Von den Meßeinrichtungen mißt eine erste Meßeinrichtung die Position der einfallenden Laserstrahlen bezogen auf eine vorbestimmte Position relativ zur Patientenunterlage. Die zweite Meßeinrichtung zeichnet die Position des Isozentrums relativ zu der Position der ersten Meßeinrichtung auf. Mit der erfindungsgemäßen Vorrichtung besteht also die Möglichkeit, mit der vorbestimmten Position der ersten Meßeinrichtung einen Punkt im Raum zu definieren, an dem die Ausrichtung des Laserstrahls verglichen und an dem die Ausrichtung des Behandlungsstrahls oder einer Diagnoseeinheit der Diagnosemaschine erfolgen kann. Die erfindungsgemäße Vorrichtung schafft ein präzises Werkzeug, um die korrekte Ausrichtung von Laserstrahlen und Behandlungsstrahl relativ zueinander zu ermöglichen. Insbesondere kann so eine Feinjustierung erreicht werden, die bei einer rein optischen Kontrolle nach Augenmaß nicht möglich ist.

In einer bevorzugten Ausgestaltung besitzt die erste Meßvorrichtung Zeilensensoren, die die Position der Laserstrahlen auf der Meßeinrichtung erfassen. Für die Laserstrahlen werden zur Erzeugung von Markierungen bevorzugt Linienlaser eingesetzt, wobei dann die Zeilensensoren jeweils quer zur Orientierung der von dem Linienlaser erzeugten Lichtebene ausgerichtet sind. Bevorzugt sind die Zeilensensoren orthogonal zu der von dem Linienlaser erzeugten Lichtebene ausgerichtet.

Die erste Meßeinrichtung besitzt bevorzugt mindestens drei Zeilensensoren, die paarweise in unterschiedlichen Richtungen im Raum orientiert sind. Die Ausrichtung in drei Raumrichtungen der Zeilensensoren erlaubt eine dreidimensionale Festlegung des vorbestimmten Punkts anhand der auf die Zeilensensoren fallenden Laserstrahlen.

Zweckmäßigerweise sind die Zeilensensoren der ersten Meßeinrichtung mit einer Auswerteeinheit verbunden, die bestimmt, ob die Laserstrahlen sich in der vorbestimmten Position relativ zu der Patientenunterlage schneiden.

In einer bevorzugten Ausgestaltung ist die Auswerteeinheit ausgelegt, um zur Justierung der Laserstrahlen relativ zu der vorbestimmten Position anzuzeigen, welches Lasergerät in welcher Richtung von der korrekten Position abweicht. In einer Weiterbildung sind die Laser mit Mitteln zum Verstellen der Position der Laser ausgestattet, wobei die Auswerteeinheit Steuersignale zur automatischen Ausrichtung der Laser in die gewünschte Position an die Verstellmittel weiterleitet.

In einer zweckmäßigen Ausgestaltung ist die zweite Meßeinrichtung mit einem Probekörper versehen und einer Halterung für ein Aufzeichnungsmaterial. Das Aufzeichnungsmaterial zeichnet ein Bild des Probekörpers in dem Behandlungsstrahl auf. Aufzeichnungsmaterial und Probekörper sind in der zweiten Meßeinrichtung angeordnet, damit der Behandlungsstrahl ein Bild des Probekörpers auf das Aufzeichnungsmaterial wirft. Bei einer Diagnosemaschine wird ein Aufzeichnungsmaterial eingesetzt für das der Diagnosestrahl ein Bild des Probekörpers erzeugt. Zusätzlich wird bei der Behandlungsmaschine und der Diagnosemaschine ein Bild des Isozentrums aufgezeichnet, so daß die Abweichung zwischen Probekörper und Isozentrum auf dem Aufzeichnungsmaterial festgehalten ist.

Zweckmäßigerweise ist der Probekörper in der zweiten Meßeinrichtung in der Position angeordnet, die in bezug auf den die erste Meßeinrichtung die Ausrichtung der Laser beschreibt.

Bevorzugt handelt es sich bei der vorbestimmten Position um die Position, die die erste Meßeinrichtung mit der Ausrichtung der Laser vergleicht und in der sich der Probekörper der zweiten Meßeinrichtung befindet, also um das erwartete Isozentrum der Maschine. Das erwartete Isozentrum der Maschine, auch als Soll-Position für das Isozentrum bezeichnet, ist der Raumpunkt, den die Steuerung der Maschine als Isozentrum des Behandlungsstrahls annimmt. Dieser Punkt muß nicht mit dem tatsächlichen Isozentrum des Behandlungsstrahls oder als Referenzpunkt annimmt. Vielmehr kann die Auswertung der zweiten Meßeinrichtung ergeben, daß erwartetes Isozentrum und tatsächliches Isozentrum auseinanderfallen. In diesem Fall sind die Ausrichtung des Behandlungsstrahls und/oder der Sollwert für das Isozentrum in der Steuerung zu ändern.

Die erfindungsgemäße Aufgabe wird ebenfalls durch ein Verfahren zur Ausrichtung von Laserstrahlen zur Anzeige einer Patientenposition relativ zu einer Maschine gelöst. Die Ausrichtung der Laserstrahlen relativ zu einem Behandlungsstrahl umfaßt hierbei sowohl die Möglichkeit, den Laserstrahl auszurichten und den Behandlungsstrahl unverändert zu lassen, als auch die Möglichkeit, den Laserstrahl unverändert zu lassen und den Behandlungsstrahl auszurichten. Auch können zur Ausrichtung beide Strahlen in ihrer Raumrichtung verändert werden, um sie in eine korrekte Position relativ zueinander zu bringen. Bei dem erfindungsgemäßen Verfahren wird eine Patientenunterlage in eine vordefinierte Referenzposition gestellt. Ein Trägerelement wird in einer definierten Position auf der Patientenunterlage angeordnet. Unter Verwendung einer ersten Meßeinrichtung wird ein Schnittpunkt der Laserstrahlen mit einer vorbestimmten Position relativ zu der Patientenunterlage zur Deckung gebracht. Unter Verwendung einer zweiten Meßeinrichtung wird das Isozentrum des Behandlungsstrahls mit der vorbestimmten Position relativ zu der Patientenunterlage zur Deckung gebracht. Sowohl die erste als auch die zweite Meßeinrichtung sind dabei auf einem Trägerelement angeordnet und die Position der Patientenunterlagen im Raum oder des Trägerelements relativ zur Patientenunterlage bleiben zwischen der Verwendung der Meßeinrichtungen unverändert. Das erfindungsgemäße Verfahren ist ein zweistufiger Vorgang, bei dem für einen im Raum festliegenden Punkt die Ausrichtung von Markierungslasern und Behandlungsstrahl überprüft werden.

In einer möglichen Ausgestaltung des erfindungsgemäßen Verfahrens erfolgt zuerst die Verwendung der zweiten Meßeinrichtung und nachfolgend eine Ausrichtung der Laser unter Verwendung der ersten Meßeinrichtung. Alternativ hierzu ist es ebenfalls möglich, zuerst die erste Meßeinrichtung zu verwenden und nachfolgend den Behandlungsstrahl unter Verwendung der zweiten Meßeinrichtung auszurichten. Dies ist besonders bevorzugt bei der Verwendung von Teilchenstrahlen, wie beispielsweise Protonenstrahlen zur Behandlung.

Die Erfindung wird nachfolgend anhand eines Ausführungsbeispiels näher erläutert.
Fig. 1 zeigt einen Patienten auf einer verstellbaren Patientenunterlage, der auf einem Behandlungstisch gelagert ist, und
Fig. 2 zeigt die erfindungsgemäße Vorrichtung mit einer ersten und zweiten Meßeinrichtung.

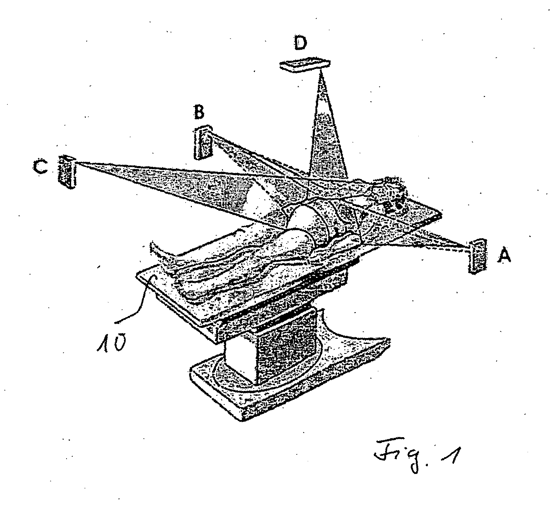

Fig. 1 zeigt eine in ihrer Position verstellbare Patientenunterlage 10, wie sie vor einem Bestrahlungsgerät installiert ist. Über die vier Linienlaser A, B, C, D wird ein fest im Raum stehendes Koordinatensystem auf den Körper eines auf der Unterlage 10 liegenden Patienten projiziert. Die Linienlaser A und B projizieren jeweils eine laterale Linie auf den Patientenkörper, Linienlaser C projiziert eine Sagitallinie und Linienlaser D projiziert eine quer über dem Thorax verlaufende Linie. Wenn die Ebenen der seitlichen Laser A und B zusammenfallen, schneiden sich sämtliche Lichtebenen in einem Punkt. Der Patient wird jedoch in der Regel an einer Reihe von Markierungen, die sich durch Projektion der Laserlinien auf den Patientenkörper ergeben, ausgerichtet. Für diese Markierungen wird der Patient auf der verfahrbaren Patientenunterlage 10 ausgerichtet und gelangt somit in eine genau spezifizierte Position relativ zu dem Therapiegerät mit seinem Behandlungsstrahl.

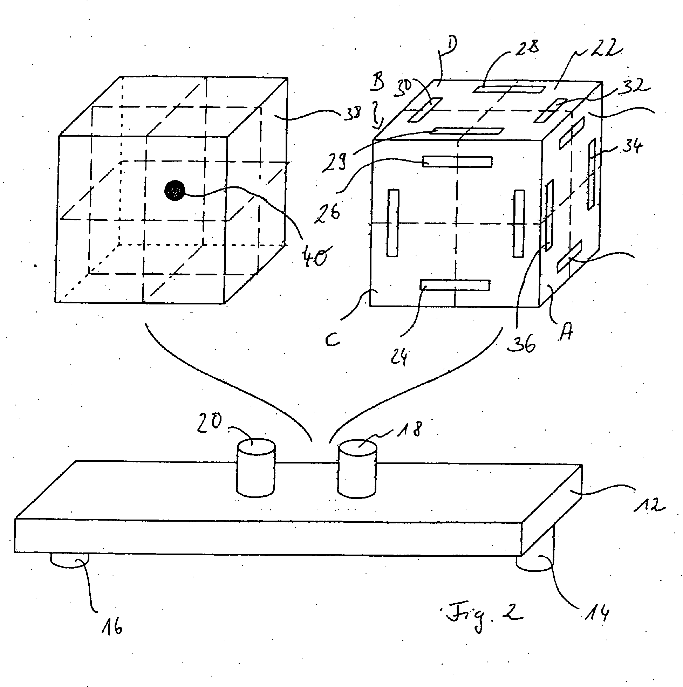

Fig. 2 zeigt die erfindungsgemäße Vorrichtung mit einem Trägerelement 12. Das Trägerelement 12 besitzt auf einer Seite zwei Verbindungselemente 14, 16, mit denen sie in einer vordefinierten Position auf der Patientenunterlage angeordnet werden kann. Die Patientenunterlage besitzt hierzu Ausnehmungen (sogenannte Notches), die die Stifte 14, 16 aufnehmen und damit die Position festlegen. Auf der gegenüberliegenden Seite der Trägerplatte sind zwei als Stifte ausgebildete Verbindungselemente 18, 20 vorgesehen. Die Verbindungselemente können unterschiedliche Durchmesser oder einen unterschiedlichen Querschnitt besitzen. Allgemein dienen Verbindungselemente dazu, das Trägerelement mit der Patientenunterlage definiert und reproduzierbar zu verbinden. Die Verbindungselemente sind zur Verbindung mit zwei Meßeinrichtungen 22, 38 vorgesehen. Die erste Meßeinheit 22 ist im wesentlichen würfelförmig aufgebaut und besitzt an wenigsten drei Seiten Zeilensensoren 24, 26, die paarweise senkrecht zueinander angeordnet sind. Die Seiten der Meßeinrichtung 22 in Fig. 2 sind entsprechend den Lasern A bis D aus Fig. 1 gekennzeichnet. Auf jeder der Seiten befinden sich jeweils zwei Paare von Zeilensensoren, so daß auch das Laserlicht von zwei Lasern erfaßt werden kann. So erfassen beispielsweise die auf der Seite D angeordneten Zeilensensoren 28 und 30 das Licht des Lasers C, während die Zeilensensoren 32 und 34 das Licht des Lasers D empfangen. Die Zeilensensoren auf der ersten Meßeinrichtung sind in der Lage, die Position zu bestimmen, in der das Laserlicht auf den Zeilensensor trifft und erlauben es so, die Position der Meßeinrichtung auf der Patientenunterlage im Raum zu bestimmen.

Streng genommen sind auf der Seite C keine Zeilensensoren notwendig und auf der Seite A und B lediglich die Zeilensensoren 34, 36 für die lateralen Laser A und B. Die redundante Messung erhöht jedoch die Genauigkeit.

Die zweite Meßeinrichtung 38 dient zur Bestimmung der Position des Behandlungsstrahls. Zur Bestimmung der Strahlenposition ist in der Meßeinrichtung 38 ein Probekörper 40 angeordnet, der mit dem Strahl wechselwirkt. Beispielsweise kann der Probekörper 40 eine größere Dichte besitzen als das übrige Material der Meßeinrichtung, so daß auf einem Abbildungsmedium (nicht dargestellt) ein Schatten des Probekörpers 40 entsteht. Das Aufzeichnungsmedium ist in der zweiten Meßeinrichtung 38 ferner derart angeordnet, daß das Isozentrum auf diesem abgebildet wird. Hierdurch kann durch Auswertung des Abbildungsmediums sichergestellt werden, daß der Abstand zwischen Probekörper 40 und Isozentrum gemessen wird.

Die Meßeinrichtung 38 erlaubt, die Position des Isozentrums relativ zu dem Probekörper 40 zu bestimmen. Die Meßeinrichtung 22 erlaubt, den Schnittpunkt der Lichtebenen räumlich exakt festzulegen. Auf diese Weise kann die Position des Isozentrums mit dem Schnittpunkt der Laser verglichen werden und so eine Ausrichtung von Linienlaser und Behandlungsstrahl relativ zueinander erfolgen. Die Meßeinrichtung 22 und 38 werden hierzu nacheinander auf die Trägerplatte 12 gesetzt.

## Patentansprüche

1. Vorrichtung zur Überprüfung der Ausrichtung von Laserstrahlen (A, B, C, D) zur Anzeige einer Position relativ zu einer Diagnose- und/oder Therapiemaschine, die ein Isozentrum aufweist, wobei die Maschine eine in ihrer Position verstellbaren Patientenunterlage (10) besitzt, Laserstrahlen und Isozentrum sind relativ zueinander derart ausgerichtet, daß die Laserstrahlen sich in dem Isozentrum der Maschine schneiden, wobei die Vorrichtung folgendes aufweist:
- ein Trägerelement (12), das mit Mitteln (14, 16) versehen ist, um in einer vorbestimmten Position an der Patientenunterlage (10) der Maschine angeordnet zu werden,
- eine erste und zweite Meßeinrichtung (22, 38), die jeweils mit Verbindungsmitteln für das Trägerelement (12) ausgestattet sind, wobei
- die erste Meßeinrichtung (22) die Position der einfallenden Laserstrahlen bezogen auf eine vorbestimmte Position relativ zu der Patientenunterlage (10) mißt, die zweite Meßeinrichtung (38) die Position des Isozentrums bezogen auf die vorbestimmte Position der ersten Meßeinrichtung aufzeichnet,
- wobei die erste und zweite Meßeinrichtung dazu ausgebildet sind, nacheinander auf das Trägerelement (12) gesetzt zu werden, und
- wobei die zweite Meßeinrichtung einen Probekörper und ein Aufzeichnungsmaterial besitzt, das zum Aufzeichnen eines Bildes des Probekörpers in dem Behandlungsstrahl geeignet ist, wobei die zweite Meßeinrichtung eine Halterung für das Aufzeichnungsmaterial besitzt.

2. Vorrichtung nach Anspruch 1, **dadurch gekennzeichnet, daß** die erste Meßeinrichtung (22) Zeilensensoren (24, 26, 30, 32, 34, 36) besitzt, die die Position der Laserstrahlen auf der ersten Meßeinrichtung erfassen.

3. Vorrichtung nach Anspruch 1 oder 2, **dadurch gekennzeichnet, daß** die erste Meßeinrichtung (22) würfelförmig aufgebaut ist und an mindestens drei Seiten Zeilensensoren aufweist, die paarweise senkrecht zueinander angeordnet sind.

4. Vorrichtung nach Anspruch 2 oder nach den Ansprüchen 2 und 3, **dadurch gekennzeichnet, daß** die Zeilensensoren der ersten Meßeinrichtung mit einer Auswerteeinheit verbunden sind, die bestimmt, ob sich die Laserstrahlen in der vorbestimmten Position relativ zu der Patientenunterlage schneiden.

5. Vorrichtung nach Anspruch 4, **dadurch gekennzeichnet, daß** die Auswerteeinheit zur Justierung der Laser in eine vorbestimmte Position anzeigt, welcher Laser in welche Richtung von der Position abweicht.

6. Vorrichtung nach Anspruch 5, **dadurch gekennzeichnet, dass** die Laser jeweils mit Mitteln zur Verstellung ihrer Position ausgestattet sind und die Auswerteeinheit Steuersignale erzeugt, um die Laser in eine gewünschte Position auszurichten.

7. Vorrichtung nach einem der Ansprüche 1 bis 6, **dadurch gekennzeichnet, daß** der Probekörper (40) in der vorbestimmten Position relativ zu der Patientenunterlage (10) in der zweiten Meßeinrichtung (38) angeordnet ist.

8. Vorrichtung nach einem der Ansprüche 1 bis 7, **dadurch gekennzeichnet, daß** das Aufzeichnungsmaterial in der zweiten Meßeinrichtung (38) derart gehalten ist, daß ein Bild von dem Probekörper (40) und dem Isozentrum des Behandlungsstrahls aufgezeichnet wird.

9. Vorrichtung nach einem der Ansprüche 1 bis 8, **dadurch gekennzeichnet, daß** die vorbestimmte Position relativ zu der Patientenunterlage dem erwarteten Isozentrum der Therapiemaschine entspricht.

10. Verfahren zur Ausrichtung von Laserstrahlen zur Anzeige einer Position relativ zu einer Diagnose- und/oder Therapiemaschine mit folgenden Schritten:
- Eine Patientenunterlage wird in eine definierte Referenzposition gestellt,
- ein Trägerelement wird in einer definierten Position auf der Patientenunterlage angeordnet,
- unter Verwendung einer ersten Meßeinrichtung (22) wird ein Schnittpunkt der Laserstrahlen mit einer vorbestimmten Position relativ zu der Patientenunterlage zur Deckung gebracht,
- unter Verwendung einer zweiten Meßeinrichtung (38) wird das Isozentrum des Behandlungsstrahls mit der vorbestimmten Position relativ zu der Patientenunterlage zur Deckung gebracht, wobei die zweite Meßeinrichtung einen Probekörper und ein Aufzeichnungsmaterial besitzt, das ein Bild des Probekörpers in dem Behandlungsstrahl aufzeichnet, wobei die zweite Meßeinrichtung eine Halterung für das Aufzeichnungsmaterial besitzt,
- wobei die erste und zweite Messeinrichtung nacheinander auf das Trägerelement (12) gesetzt werden, und
- wobei die erste und zweite Meßeinrichtung auf dem Trägerelement angeordnet sind und die Position der Patientenunterlage im Raum oder des Trägerelements relativ zu der Patientenunterlage zwischen der Verwendung der Meßeinrichtungen unverändert bleibt.

11. Verfahren nach Anspruch 10, **dadurch gekennzeichnet, daß** zuerst die zweite Meßeinrichtung verwendet und nachfolgend die Laser unter Verwendung der ersten Meßeinrichtung ausgerichtet werden.

12. Verfahren nach Anspruch 10, **dadurch gekennzeichnet, daß** zuerst die erste Meßeinrichtung verwendet wird und nachfolgend der Behandlungsstrahl und/oder Diagnosemittel unter Verwendung der zweiten Meßeinrichtung ausgerichtet wird.

## Claims

1. An apparatus for checking the alignment of laser beams (A, B, C, D) for indicating a position in relation to a diagnostic and/or therapeutic machine, which has a isocentre, wherein the machine has a patient support (10) movable in its position, laser beams and isocentre being aligned in relation to each other such that the laser beams intersect with each other in the isocentre of the machine, wherein the machine has the following:
- a carrier element (12), which is provided with means (14, 16) for being arranged in a predetermined position on the patient support (10) of the machine,
- a first and a second measuring device (22, 38), each one at a time being equipped with connection means for the carrier element (12), wherein
- the first measuring device (22) measures the position of the impingent laser beams with respect to a predetermined position in relation to the patient support (10), the second measuring device (38) records the position of the isocentre in relation to the predetermined position of the first measuring device.
- wherein the first and the second measuring device are designed to be put onto the carrier plate (12) one after the other, and
- wherein the second measuring device has a sample body and a recording material recording which is adapted for recording an image of the sample body in the therapeutic beam, wherein the second measuring device has a holder for the recording material.

2. An apparatus according to claim 1, **characterised in that** the first measuring device (22) has line sensors (24, 26, 30, 32, 34, 36), which acquire the position of the laser beams on the first measuring device.

3. An apparatus according to claim 1 or 2, **characterised in that** the first measuring device (22) is designed as being cube-like and has line sensors on at least three sides, which are arranged pair wise vertically with respect to each other.

4. An apparatus according to claim 2 or to claim 2 and 3, **characterised in that** the line sensors of the first measuring device are connected to an analysing unit, which determines whether the laser beams intersect with each other in the predetermined position with respect to the patient support.

5. An apparatus according to claim 4, **characterised in that** the analysing unit for adjusting the lasers into a predetermined position indicates which laser deviates into which direction from that position.

6. An apparatus according to claim 5, **characterised in that** the lasers are each one at a time equipped with means for moving their position, and the analysing unit generates control signals for aligning the lasers into a desired position.

7. An apparatus according to any one of claims 1 to 6, **characterised in that** the sample body (40) is arranged in the second measuring device (38) in the predetermined position in relation to the patient support (10).

8. An apparatus according to any one of claims 1 to 7, **characterised in that** the recording material in the second measuring device (38) is held such that an image of the sample body (40) and of the isocentre of the therapeutic beam is recorded.

9. An apparatus according to any one of claims 1 to 8, **characterised in that** the predetermined position in relation to the patient support corresponds to the expected isocentre of the therapeutic machine.

10. A method for the alignment of laser beams for indicating a position in relation to a diagnostic and/or therapeutic machine, with the following steps:
- a patient support is set into a defined reference position,
- a carrier element is arranged in a defined position on the patient support,
- using a first measuring device (22), a point of intersection of the laser beams is brought into coincidence with a predetermined position in relation to the patient support,
- using a second measuring device (38), the isocentre of the therapeutic beam is brought into coincidence with the predetermined position in relation to the patient support, wherein the second measuring device has a sample body and a recording material recording an image of the sample body in the therapeutic beam, wherein the second measuring device has a holder for the recording material,
- wherein the first and second measuring device are put onto the carrier plate (12) one after the other, and
- wherein the first and the second measuring device are arranged on the carrier element and the position of the patient support in space, or of the carrier element in relation to the patient support, remains unchanged between the usage of the measuring devices.

11. A method according to claim 10, **characterised in that** the second measuring device is used at first, and subsequently the lasers are aligned by using the first measuring device.

12. A method according to claim 10, **characterised in that** the first measuring device is used at first, and subsequently the therapeutic beam and/or the diagnostic beam are aligned using the second measuring device.

## Revendications

1. Dispositif de surveillance du positionnement de faisceaux laser (A, B, C, D) pour indiquer une position par rapport à un appareil de diagnostic et/ou de thérapie, qui présente un isocentre, l'appareil possédant un support de patient (10) dont la position est réglable, les faisceaux laser et l'isocentre étant positionnés les uns par rapport aux autres de telle façon que les faisceaux laser se recoupent dans l'isocentre de l'appareil, le dispositif présentant :
- un élément porteur (12), qui est pourvu de moyens (14, 16) pour être disposé dans une position prédéfinie au niveau du support de patient (10) de l'appareil,
- un premier et un second dispositifs de mesure (22, 38), qui sont équipés respectivement de moyens de raccordement pour l'élément porteur (12),
- le premier dispositif de mesure (22) mesurant la position des faisceaux laser incidents sur la base d'une position prédéfinie par rapport au support de patient (10), le second dispositif de mesure (38) enregistrant la position de l'isocentre sur la base de la position prédéfinie du premier dispositif de mesure,
- les premier et second dispositifs de mesure étant conçus pour être placés l'un après l'autre sur l'élément porteur (12), et
- le second dispositif de mesure possédant un spécimen d'essai et un matériau d'enregistrement qui est adapté pour l'enregistrement d'une image du spécimen d'essai dans le faisceau de traitement, le second dispositif de mesure possédant un support pour le matériau d'enregistrement.

2. Dispositif selon la revendication 1, **caractérisé en ce que** le premier dispositif de mesure (22) possède des capteurs linéaires (24, 26, 30, 32, 34, 36) qui captent la position des faisceaux laser sur le premier dispositif de mesure.

3. Dispositif selon la revendication 1 ou 2, **caractérisé en ce que** le premier dispositif de mesure (22) est construit en forme de cube et présente sur au moins trois côtés des capteurs linéaires qui sont disposés par paires perpendiculairement l'un par rapport à l'autre.

4. Dispositif selon la revendication 2 ou selon les revendications 2 et 3, **caractérisé en ce que** les capteurs linéaires du premier dispositif de mesure sont raccordés à une unité d'évaluation qui détermine si les faisceaux laser dans la position prédéfinie par rapport au support de patient se recoupent.

5. Dispositif selon la revendication 4, **caractérisé en ce que** l'unité d'évaluation pour l'ajustage des lasers dans une position prédéfinie indique quel laser diverge de la position et dans quelle direction.

6. Dispositif selon la revendication 5, **caractérisé en ce que** les lasers sont équipés respectivement de moyens pour le réglage de leur position et l'unité d'évaluation génère des signaux de commande pour positionner les lasers dans une position souhaitée.

7. Dispositif selon l'une des revendications 1 à 6, **caractérisé en ce que** le spécimen d'essai (40) dans la position prédéfinie par rapport au support de patient (10) est disposé dans le second dispositif de mesure (38).

8. Dispositif selon l'une des revendications 1 à 7, **caractérisé en ce que** le matériau d'enregistrement est supporté dans le second dispositif de mesure (38) de telle façon qu'une image du spécimen d'essai (40) et de l'isocentre du faisceau de traitement est enregistrée.

9. Dispositif selon l'une des revendications 1 à 8, **caractérisé en ce que** la position prédéfinie par rapport au support de patient correspond à l'isocentre prévu de l'appareil de thérapie.

10. Procédé de positionnement de faisceaux laser pour indiquer une position par rapport à un appareil de diagnostic et/ou de thérapie avec les étapes suivantes :
- un support de patient est placé dans une position de référence définie,
- un élément porteur est disposé dans une position définie sur le support de patient,
- au moyen d'un premier dispositif de mesure (22) un point d'intersection des faisceaux laser est fait concorder avec une position prédéfinie par rapport au support de patient,
- au moyen d'un second dispositif de mesure (38), l'isocentre du faisceau de traitement est fait concorder avec la position prédéfinie par rapport au support de patient, le second dispositif de mesure possédant un spécimen d'essai et un matériau d'enregistrement qui enregistre une image du spécimen d'essai dans le faisceau de traitement, le second dispositif de mesure possédant un support pour le matériau d'enregistrement,
- les premier et second dispositifs de mesure étant placés sur l'élément porteur (12) l'un après l'autre, et
- les premier et second dispositifs de mesure étant disposés sur l'élément porteur et la position du support de patient dans la pièce ou de l'élément porteur par rapport au support de patient restant inchangée entre l'utilisation des dispositifs de mesure.

11. Procédé selon la revendication 10, **caractérisé en ce que** le second dispositif de mesure est d'abord utilisé et ensuite les lasers sont positionnés au moyen du premier dispositif de mesure.

12. Procédé selon la revendication 10, **caractérisé en ce que** le premier dispositif de mesure est d'abord utilisé et ensuite le faisceau de traitement et/ou le moyen de diagnostic est positionné au moyen du second dispositif de mesure.
